## Europäisches Patentamt

## European Patent Office

## Office européen des brevets

(11) Numéro de publication: **0 178 442**
**B1**

## (12) FASCICULE DE BREVET EUROPÉEN

(45) Date de publication du fascicule du brevet:
16.03.88

(21) Numéro de dépôt: 85111161.7

(22) Date de dépôt: 04.09.85

(51) Int. Cl.⁴: **C 11 C 1/00,** C 07 C 57/12,
C 07 C 51/42, C 07 C 51/487

(54) Procédé d'enrichissement sélectif en acides gras polyinsaturés delta-6 d'un mélange contenant des acides gras delta-6 et delta-9, fractions enrichies obtenues et leur utilisation.

(30) Priorité: 10.10.84 CH 4858/84

(43) Date de publication de la demande:
23.04.86 Bulletin 86/17

(45) Mention de la délivrance du brevet:
16.03.88 Bulletin 88/11

(84) Etats contractants désignés:
AT BE DE FR IT LU NL SE

(56) Documents cité:
EP-A-0 092 076
US-A-2 596 344
US-A-2 662 879

(73) Titulaire: **SOCIETE DES PRODUITS NESTLE S.A.,**
**Case postale 353, CH- 1800 Vevey (CH)**

(72) Inventeur: **Traitler, Helmut, Route de St- Légier**
**10a, CH- 1800 Vevey (CH)**
Inventeur: **Studer, Alfred, Ch. Entre- deux- Villes 5,**
**CH- 1802 Corseaux (CH)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1988

## Description

L'invention concerne l'enrichissement sélectif en acides gras polyinsaturés dont la première double liaison est en position 6, notamment en acide gamma-linolénique d'un mélange contenant des acides gras polyinsaturés dont la première double liaison est en position 6 ou 9.

On connaît l'importance biologique de l'acide gamma-linolénique (6,9,12-octadécatriénoïque ($\Delta$6, $\omega$6)). C'est un intermédiaire essentiel dans le processus métabolique intervenant dans un organisme vivant à partir de l'acide linoléique (9,12-octadécadiénoïque ($\Delta$9, $\omega$6)) et aboutissant par l'intermédiaire de l'acide dihomo-gamma-linolénique (5,8,11-eicosatriénoïque) à l'acide arachidonique (5,8,11,14-eicosatétraénoïque). Cette propriété est mise à profit dans ses nombreuses applications diététiques, cosmétiques et pharmaceutiques. On sait aussi que l'acide alpha-linolénique (9,12,15-octadécatriénoïque ($\Delta$9, $\psi$3)) n'intervient pas de la même manière dans ce processus métabolique.

La plupart des huiles végétales contiennent les acides polyinsaturés 49 linoléique ($\omega$6) et alpha-linolénique ($\omega$3). Une source d'acide polyinsaturé $\Delta$6 ($\omega$6) gamma-linolénique très intéressante est constituée par les pépins de fruits du genre Ribes, en particulier de cassis. L'huile des pépins de ces fruits contient des quantités appréciables d'acides polyinsaturés $\Delta$9 linoléique ($\omega$6) et alpha-linolénique ($\omega$3). A titre indicatif, l'huile des pépins de ces fruits est constituée de triglycérides des acides gras suivants, en pourcentage pondéral:

| Acides gras | Cassis | Groseille | Groseille à maquereau |
|---|---|---|---|
| C16:0 | 6-7 | 4-5 | 7-6 |
| C18:0 | 1-2 | 1-2 | 1 |
| C18:1 cis | 9-10 | 14-15 | 15-16 |
| C18:1 trans | 0,5 | 0,5-1 | 1-2 |
| C18:2,$\Delta$9,12 | 47-49 | 41-42 | 39-41 |
| C18:3,$\Delta$6,9,12 | 15-19 | 4-5 | 10-12 |
| C18:3,$\Delta$9,12,15 | 12-14 | 29-31 | 19-20 |
| C18:4,$\Delta$6,9,12,15 | 3-4 | 2,5-3,5 | 4-5 |

Pour certaines applications, en particulier pharmaceutiques, on désire enrichir sélectivement le mélange d'acides gras provenant de ces huiles en polyinsaturés $\Delta$6, en particulier en acide gamma-linolénique. Or, la méthode classique de fractionnement des acides gras par chromatographie liquide haute performance (HPLC) ne permet pas de séparer l'acide gamma-linolénique de l'acide alpha-linolénique.

Nous avons trouvé de manière surprenante qu'on pouvait séparer ces deux isomères par une méthode simple appliquée jusqu'à présent pour séparer les acides gras de différents degrés de saturation. En particulier la séparation des acides gras insaturés des acides gras saturés est décrite, par exemple dans le brevet français No. 1 603 383, correspondant au britannique No. 1 240 513, qui concerne l'enrichissement en acide gamma-linolénique d'un mélange d'acides gras provenant de l'huile d'onagre (Oenothera) qui ne contient par ailleurs pratiquement pas d'acide alpha-linolénique.

Le procédé selon la présente invention est caractérisé par le fait qu'on traite le mélange avec l'urée en solution dans un alcanol inférieur dans un rapport pondéral acides gras autres que les polyinsaturés $\Delta$6: urée de 1:2,0 à 1:4,5, qu'on sépare le complexe d'inclusion insoluble formé et qu'on recueille dans la phase liquide une fraction enrichie en acides gras polyinsaturés $\Delta$6, en particulier en acide gamma-linolénique.

Pour la mise en oeuvre du procédé on peut utiliser de préférence un mélange d'acides gras obtenu par saponification d'une matière première contenant de l'huile de pépins de fruits du genre Ribes, de préférence l'huile de cassis (Ribes nigrum). Ainsi, on peut saponifier l'huile brute ou raffinée ou en variante traiter directement les pépins, de préférence mis sous forme de flocons. La saponification s'effectue de manière classique en traitant la matière première par une base forte concentrée, par exemple l'hydroxyde de sodium, de préférence en milieu hydro-alcoolique chaud, contenant avantageusement un agent séquestrant des ions métalliques comme par exemple l'éthylènediaminetétraacétate disodique, en séparant les insaponifiables par un solvant, par exemple, l'hexane et en neutralisant la phase aqueuse, par exemple par l'acide chlorhydrique en solution aqueuse concentrée. Après la saponification, le mélange obtenu peut, de préférence, être protégé contre l'oxydation par addition d'un antioxydant, par exemple 100 à 600 ppm (parties par million) de gallate de propyle ou de préférence 200 à 400 ppm de palmitate d'ascorbyle.

On peut également utiliser comme produit de départ les savons obtenus lors de la neutralisation de l'huile brute au cours du raffinage.

Le fractionnement consiste à opérer dans des conditions permettant la formation sélective d'un complexe des acides gras autres que $\Delta$6 avec l'urée dans un milieu tel que l'urée soit soluble mais pas les complexes d'inclusion formés. Un milieu approprié est constitué par un alcanol inférieur, c'est-à-dire de 1 à 4 atomes de carbone, de préférence l'éthanol ou le méthanol, ce dernier étant particulièrement approprié par suite de son grand pouvoir de dissolution de l'urée. La solution sera de préférence saturée et contiendra 45-50 % en poids d'urée. On préparera avantageusement la solution saturée par dissolution de l'urée dans le méthanol par exemple à environ 60°C sous agitation jusqu'à obtenir une solution transparente.

La quantité d'urée est proportionnelle à la quantité totale d'acides gras à éliminer du mélange. Lorsque le mélange d'acides gras provient de la saponification d'une matière première mentionnée ci-dessus, on utilisera un rapport pondéral matière première: urée de préférence d'environ 1:3. La quantité de méthanol utilisée sera avantageusement 2 à 6 fois et de préférence environ 3 fois la quantité pondérale de matière première mise en oeuvre. Après agitation vigoureuse du mélange, on le refroidit à une température de 0 à 12°C et de préférence à 4-6°C pendant 10-20 h. Après séparation du précipité, par exemple par centrifugation, on neutralise l'urée n'ayant pas réagi en traitant la solution par un acide, par exemple l'acide chlorhydrique en solution aqueuse, de préférence concentrée et on extrait les acides gras par un solvant, de préférence l'hexane puis on élimine celui-ci, de préférence par évaporation sous vide.

Dans une forme d'exécution préférée, on traite la fraction enrichie ci-dessus une nouvelle fois par l'urée, dans un rapport pondéral acides gras totaux:urée de 1:1,4 à 1:1,6. On obtient de cette manière une fraction contenant 92 à 96 % en poids d'acides gras polyinsaturés Δ6 dont 77 à 81 % d'acide gamma-linolénique.

Si on le désire, on peut préparer l'acide gamma-linolénique pratiquement pur à partir de la fraction provenant du second fractionnement à l'urée par chromatographie liquide haute performance en phase inversée, ce qui permet de séparer l'acide gamma-linolénique de l'acide stéaridonique (C18:4,Δ6,9,12,15).

En variante, on peut procéder à la séparation chromatographique d'un mélange contenant les acides gras provenant de la saponification et ensuite effectuer le fractionnement à l'urée de ce mélange comme indiqué ci-dessus.

Les fractions d'acides gras libres obtenues selon l'invention peuvent être utilisées dans toutes les applications de l'acide gamma-linolénique telles quelles ou sous forme d'huile obtenue par recombinaison avec le glycérol, par exemple dans les compositions nutritives ou les médicaments décrits dans les demandes de brevet européen publiées Nos. 92.085 et 92.076 et administrées par voie orale, entérale, parentérale ou topique.

Les fractions recombinées avec le glycérol sous forme d'huile conviennent en particulier pour l'application topique en dermatologie et en cosméto-dermatologie. Les fractions contenant l'acide gamma-linolénique pratiquement pur peuvent également servir de produit de départ dans la synthèse de l'acide dihomo-gamma-linolénique.

Les exemples ci-après illustrent l'invention. Dans ceux-ci les pourcentages et parties sont en poids, sauf indication contraire.

## Exemple 1

On ajoute à 30 kg d'huile de cassis raffinée et désodorisée 63,9 kg d'une, solution hydro-éthanolique à 14,2 % d'hydroxyde de sodium contenant 95 g d'éthylènediaminetétraacétate disodique. On chauffe le mélange à 60°C et on le maintient à cette température pendant 30 min. sous agitation. On ajoute ensuite 12 kg d'eau et on refroidit la solution à 30°C.

Après addition de 79 kg d'hexane et agitation 1 h. à 30°C on laisse le mélange décanter pendant 15 min. et on élimine la phase supérieure contenant les insaponifiables. On ajoute alors 30 kg d'acide chlorhydrique à 32 % à la phase inférieure sous agitation (jusqu'à pH 1) en s'assurant que la température ne dépasse pas 30°C. Après décantation, on élimine la phase inférieure et on concentre la phase supérieure sous le vide de la trompe à eau à 40°C.

On ajoute les 28,5 kg du mélange d'acides gras obtenu sous agitation constante à une solution saturée claire de 90 kg d'urée dans 190 kg de méthanol à 60°C. On refroidit le mélange à 5°C et on le maintient à cette température pendant 15 h. On essore ensuite la phase solide apparue par centrifugation et on laisse reposer la phase liquide pendant 4 h à 5°C. On sépare à nouveau la phase liquide de la phase solide par essorage. On recueille ainsi 180 kg de phase liquide. On ajoute à celle-ci sous agitation 45,9 kg d'hexane, 39,9 kg d'une solution aqueuse d'acide chlorhydrique à 32 % et 106 kg d'eau et on porte le mélange à 30°C. Après agitation pendant 1 h à 30°C, on laisse au repos pendant 10 min., puis on recueille la phase supérieure par décantation. On extrait ensuite la phase inférieure avec 12 kg d'hexane sous agitation pendant 15 min. à 30°C. On laisse reposer le mélange pendant 15 min. puis on recueille la phase supérieure par décantation, qu'on combine avec la phase supérieure précédente. Aux phases combinées précédentes on mélange 50 kg d'eau sous agitation vive. On laisse reposer ensuite le mélange 3 h à température ambiante, on sépare la phase supérieure contenant les acides gras et on la sèche par évaporation sous le vide de la trompe à eau à 40°C. On obtient ainsi 6,82 kg d'acides gras (rendement, 22,7 % par rapport à l'huile engagée). Le mélange a la composition pondérale ci-après déterminée par chromatographie en phase gazeuse:

|  | % |
|---|---|
| C18:1 | 0,6 |
| C18:2,Δ9,12 | 22,1 |
| C18:3,Δ6,9,12 | 55,6 |
| C18:3,Δ9,12,15 | 10,7 |
| C18:4,Δ6,9,12,15 | 11,0 |

# 0 178 442

**Exemple comparatif**

On procède au fractionnement des acides gras de l'huile de cassis mise en oeuvre ci-dessus par chromatographie préparative liquide haute performance sous forme de leurs esters méthyliques, sur des colonnes RP-18 à phase inversée.

L'élution d'un échantillon de concentration 20 % dans le mélange solvant: 67,5 % méthanol/22,5 % éthanol/10 % eau, à un débit de 150 ml/min. conduit aux fractions suivantes:

1) une première fraction représentant 7,6 % du mélange, contenant 52 % d'acide stéaridonique, C18:4,$\Delta$6,9,12,15;

2) une seconde fraction représentant 25,4 % du mélange, contenant 41 % d'acide alpha-linolénique, C18:3,$\Delta$9,12,15 et 43 % d'acide gamma-linolénique, C18:3,$\Delta$6,9,12;

3) une troisième fraction représentant 45,8 % du mélange, contenant 87 % d'acide linoléique, C18:2,$\Delta$9,12 et

4) une quatrième fraction représentant 21,2 % du mélange, contenant 85 % d'acides gras saturés.

La comparaison des rapports des quantités d'acide gamma-linolénique et d'acide alpha-linolénique calculés pour le mélange obtenu selon l'exemple 1, pour la fraction 2) de l'exemple comparatif et pour l'huile de cassis donne une indication de la sélectivité du procédé d'enrichissement selon l'invention:

|  | mélange obtenu selon l'exmple 1 | huile ce cassis | fraction 2) ci-dessus |
|---|---|---|---|
| % acide gamma-linolénique / % acide alpha-linolénique | 5,2 | 1,3 | 1,1 |

On constate à partir des valeurs indiquées ci-dessus que, contrairement au procédé selon l'invention, la méthode habituellement utilisée de chromatographie ne permet pas de séparer les acides gamma- et alpha-linolénique d'un mélange les contenant et donc d'enrichir le mélange en acide gamma-linolénique.

**Exemple 2**

On chauffe un mélange de 10,2 kg d'urée et 21,5 kg de méthanol à 60°C jusqu'à obtenir une solution saturée claire puis on y ajoute sous agitation les 6,82 kg de mélange d'acides gras provenant du fractionnement selon l'exemple 1. On refroidit ensuite le mélange à 5°C et on le laisse à cette température pendant 15 h. On essore alors la phase solide formée par centrifugation et on laisse reposer la phase liquide à 5°C pendant 4 h. On essore à nouveau la phase liquide et on élimine les cristaux formés.

Aux 23 kg de solution obtenue on ajoute 5,9 kg d'hexane, 5,1 kg d'une solution aqueuse d'acide chlorhydrique à 32 % et 13,5 kg d'eau. On agite vigoureusement le mélange porté à 30°C pendant 15 min. On laisse ensuite décanter pendant 10 min. et on sépare la phase supérieure contenant les acides gras. On ajoute alors 3 kg d'hexane à la phase inférieure et on agite vigoureusement le mélange pendant 15 min. On laisse reposer le mélange pendant 15 min., puis on recueille la phase supérieure par décantation, qu'on joint à la phase supérieure précédente.

On mélange les phases combinées ci-dessus avec 30 kg d'eau sous agitation pendant 15 min. On laisse le mélange au repos pendant 3 h, puis on élimine la phase inférieure par décantation et on recueille la phase supérieure qu'on sèche par évaporation sous le vide de la trompe à eau à 40°C. On ajoute alors 400 ppm (parties par million) de palmitate d'ascorbyle aux 4,05 kg de mélange d'acides gras obtenu (rendement 13,5 % par rapport au mélange d'acides gras engagé).

Celui-ci a la composition pondérale ci-après déterminée par chromatographie en phase gazeuse:

|  | % |
|---|---|
| C18:2,$\Delta$9,12 | 2,5 |
| C18:3,$\Delta$6,9,12 | 78,6 |
| C18:3,$\Delta$9,12,15 | 2,3 |
| C18:4,$\Delta$6,9,12,15 | 16,6 |

**Exemple 3**

On met des pépins de cassis sous forme de flocons. On ajoute à 35 kg de ces flocons 150 kg d'une solution hydro-éthanolique à 14,2 % d'hydroxyde de sodium contenant 223 g d'éthylènediaminetétraacétate disodique et on agite pendant 1 h la suspension portée à 60°C, puis on sépare le résidu par filtration sous vide. On rince et on lave ensuite le filtre avec une quantité d'éthanol correspondant à 1 fois le poids des flocons mis en oeuvre, puis on évapore la majeure partie de l'éthanol sous forme de mélange azéotropique. On ajoute alors au résidu une solution aqueuse à 32 % d'acide chlorhydrique jusqu'à ce qu'on obtienne un pH de 1.

Après décantation, on sépare la plupart des acides gras sous forme de la phase organique supérieure apparue. On ajoute 35 kg d'hexane à la phase aqueuse inférieure et on décante à nouveau. On lave la phase inférieure une seconde fois avec 35 kg d'hexane. On lave alors les phases organiques combinées (comprenant les acides gras et les deux solutions dans l'hexane) deux fois avec 70 kg d'eau à chaque lavage et on évapore le solvant sous le vide de la trompe à eau à 40°C. Le résidu représente 6,72 kg d'un mélange d'acides gras de composition déterminée par chromatographie en phase gazeuse, identique à celle du mélange obtenu par saponification de l'huile mise en oeuvre à l'exemple 1. On soumet ensuite celui-ci au fractionnement dans les conditions de l'exemple 1.

**Exemple 4**

On prépare une solution à 20 % du mélange d'acides gras provenant du double fractionnement selon l'exemple 2 dans le mélange solvant: 67,5 % méthanol/22,5 % éthanol/ 10 % eau.

On injecte ensuite 10 ml de cette solution dans un appareil de chromatographie préparative liquide haute performance pourvu de colonnes de gel de silice RP-18 à phase inversée. Le mélange solvant ci-dessus est utilisé comme phase mobile au débit de 100 ml/min. et l'identification quantitative est réalisée à l'aide d'un détecteur d'indice de réfraction.

Les deux acides gras principaux gamma-linolénique et stéaridonique du mélange obtenu selon l'exemple 2 (78,6 % d'acide gamma-linolénique, 16,6 % d'acide stéaridonique) sont pratiquement séparables par la méthode ci-dessus, les deux fractions éluées obtenues ayant les compositions suivantes:

|  |  | % |
|---|---|---|
| - 1ère fraction: | C18:3,Δ6,9,12 | 49 |
| (représentant 20 % | | |
| du mélange) | C18:4,Δ6,9,12,15 | 51 |
|  |  | % |
| - 2ème fraction: | C18:3,Δ6,9,12 | 96 |
| (représentant 80 % | | |
| du mélange) | C18:3,Δ9,12,15 | 2,5 |
|  | C18:4,Δ6,9,12,15 | 1,5 |

La 2ème fraction peut servir avantageusement de produit de départ dans la synthèse de l'acide dihomo-gamma-linolénique.

**Exemple 5**

On fait réagir 100 g des mélanges d'acides gras obtenus selon les exemples 1 à 4 avec 15 g de glycérol à 210°C pendant 5 h sous une pression de 6,7 mbar (5 mm Hg). On obtient ainsi 96 g de triglycérides avec un rendement de 92 %. Les huiles obtenues conviennent pour les applications dermatologiques et cosméto-dermatologiques.

**Revendications**

1. Procédé d'enrichissement sélectif en acides gras polyinsaturés dont la première double liaison est en position 6, notamment en acide gamma-linolénique, d'un mélange contenant des acides gras polyinsaturés dont la première double liaison est en position 6 ou 9, caractérisé par le fait qu'on traite le mélange avec l'urée en solution dans un alcanol de 1 à 4 atomes de carbone dans un rapport pondéral acides gras autres que les polyinsaturés Δ:urée de 1:2,0 à 1:4,5, qu'on sépare le complexe d'inclusion insoluble formé et qu'on recueille une fraction enrichie en acides gras polyinsaturés Δ6, en particulier en acide gamma-linolénique, dans la phase liquide.

2. Procédé selon la revendication 1, caractérisé par le fait que le mélange d'acides gras de départ est obtenu par saponification de pépins de fruits du genre Ribes ou d'une huile extraite de ces pépins, notamment de pepins ou d'huile de cassis, Ribes nigrum.

3. Procédé selon la revendication 2, dans lequel le mélange d'acides gras provient du cassis, caractérisé par le fait qu'on traite la fraction enrichie en acides gras polyinsaturés Δ6 obtenue avec l'urée dans un rapport pondéral acides gras totaux:urée de 1:1,4 à 1:1,6, qu'on sépare le complexe d'inclusion insoluble formé et qu'on recueille une fraction contenant 92 à 96 % en poids d'acides gras polyinsaturés Δ6 dont 77 à 81 % d'acide gamma-linolénique.

4. Procédé selon la revendication 3, caractérisé par le fait qu'on sépare l'acide gamma-linolénique pratiquement pur du mélange d'acides gras polyinsaturés Δ6 par chromatographie liquide haute performance en phase inversée.

5. Procédé selon l'une des revendications 1 à 3, caractérisé par le fait qu'on estérifie le mélange enrichi en acides gras polyinsaturés Δ6 avec le glycérol de manière à obtenir une huile enrichie en acides gras polyinsatures Δ6, notamment en acide gamma-linolénique.

6. Fraction ou huile contenant l'acide gamma-linolénique obtenue par le procédé selon l'une des revendications 3 à 5.

7. Composition contenant une huile selon la revendication 6.

8. Utilisation d'une fraction obtenue selon la revendication 4 comme produit de départ dans la synthèse de l'acide dihomo-gamma-linolénique.

## Patentansprüche

1. Verfahren zur selektiven Anreicherung eines mehrfach ungesättigte Fettsäuren, deren erste Doppelbindung sich in der 6- oder 9-Stellung befindet, enthaltenden Gemisches an mehrfach ungesättigten Fettsäuren, deren erste Doppelbindung sich in der 6-Stellung befindet, insbesondere an γ-Linolensäure, dadurch gekennzeichnet, daß man das Gemisch mit Harnstoff in Lösung in einem Alkanol mit 1 bis 4 Kohlenstoffatomen bei einem Gewichtsverhältnis von Fettsäuren, die von den mehrfach ungesättigten Δ6-Säuren verschieden sind, zu Harnstoff von 1:2,0 bis 1:4,5 behandelt, den gebildeten unlöslichen Einschlußkomplex abtrennt und eine an mehrfach ungesättigten Δ6-Fettsäuren, insbesondere an γ-Linolensäure, angereicherte Fraktion in der flüssigen Phase gewinnt.

2. Verfahren nach Anspruch 1, dadurch gekennzeichnet, daß das Ausgangsgemisch der Fettsäuren durch Verseifung von Kernen von Früchten der Gattung Ribes oder eines aus diesen Kernen extrahierten Öls, insbesondere von Kernen oder von einem Öl von Cassis, Ribes nigrum, erhalten wird.

3. Verfahren nach Anspruch 2, worin das Fettsäurengemisch aus Cassis stammt, dadurch gekennzeichnet, daß man die erhaltene, an mehrfach ungesättigten Δ6-Fettsäuren angereicherte Fraktion mit Harnstoff bei einem Gewichtsverhältnis von Gesamtfettsäuren zu Harnstoff von 1:1,4 bis 1:1,6 behandelt, den gebildeten unlöslichen Einschlußkomplex abtrennt und eine Fraktion gewinnt, die 92 bis 96 Gew.% mehrfach ungesättigte Δ6-Fettsäuren, davon 77 bis 81 % γ-Linolensäure, enthält.

4. Verfahren nach Anspruch 3, dadurch gekennzeichnet, daß man die praktisch reine γ-Linolensäure durch Hochleistungsflüssigkeitschromatographie in inverser Phase aus dem Gemisch von mehrfach ungesättigten Δ6-Fettsäuren abtrennt.

5. Verfahren nach einem der Ansprüche 1 bis 3, dadurch gekennzeichnet, daß man das an mehrfach ungesättigen Δ6-Fettsäuren angereicherte Gemisch mit Glyzerin verestert, um ein an mehr ach ungesättigen Δ6-Fettsäuren, insbesondere an γ-Linolensäure, angereichertes Öl zu erhalten.

6. Fraktion oder Öl mit einem Gehalt an γ-Linolensäure, erhalten nach dem Verfahren gemäß einem der Ansprüche 3 bis 5.

7. Zusammensetzung, enthaltend ein Öl gemäß Anspruch 6.

8. Verwendung einer gemäß Anspruch 4 erhaltenen Fraktion als Ausgangsprodukt in der Synthese der Dihomo-gamma-linolensäure.

## Claims

1. A process for the selective enrichment with polyunsaturated fatty acids, of which the first double bond is in the 6 position, particularly with γ-linolenic acid, of a mixture containing polyunsaturated fatty acids of which the first double bond is in the 6 or 9 position, characterized in that the mixture is treated with urea dissolved in a lower alkanol in a ratio by weight of fatty acids other than the Δ6 polyunsaturated to urea of from 1:2.0 to 1:4.5, in that the insoluble inclusion complex formed is separated and in that a fraction enriched with Δ6 polyunsaturated fatty acids, particularly with γ-linolenic acid, is collected in the liquid phase.

2. A process as claimed in Claim 1, characterized in that the starting mixture of fatty acids is obtained by saponification of seeds of fruit of the genus Ribes or of an oil extracted therefrom, more particularly seeds or oil of black currant, Ribes nigrum.

3. A process as claimed in Claim 2, in which the mixture of fatty acids originates from black currant, characterized in that the fraction enriched with Δ6 polyunsaturated fatty acids obtained is treated with urea in a ratio by weight of total fatty acids to urea of from 1:1.4 to 1:1.6, in that the insoluble inclusion complex formed is separated and in that a fraction containing from 92 to 96 % by weight of Δ6 polyunsaturated fatty acids, of which 77 to 81 % consist of γ-linolenic acid, is collected.

4. A process as claimed in Claim 3, characterized in that the substantially pure γ-linolenic acid is separated from the mixture of Δ6 polyunsaturated fatty acids by reverse-phase high-performance liquid chromatography.

5. A process as claimed in any of Claims 1 to 6, characterized in that the mixture enriched with Δ6 polyunsaturated fatty acids is esterified with glycerol to obtain an oil enriched with Δ6 polyunsaturated fatty acids, more especially with γ-linolenic acid.

6. A fraction or oil containing the γ-linolenic acid obtained by the process claimed in any of Claims 3 to 5.

7. A composition containing the oil claimed in Claim 6.

8. The use of a fraction obtained in accordance with Claim 4 as starting product in the synthesis of dihomo-γ-linolenic acid.